# EUROPEAN PATENT APPLICATION

(11) **EP 4 328 302 A1**
(43) Date of publication of application: **28.02.2024**
(21) Application number: 22192384.0
(22) Date of filing: 26.08.2022
(51) Int. Cl.: C12N 9/02, C12P 21/02

(54) **SYNTHESIS OF FUNCTIONAL CYTOCHROME P450 MONOOXYGENASES USING A EUKARYOTIC CELL-FREE SYSTEM COMPRISING MEMBRANE VESICLES**

(71) Applicant: Fraunhofer-Gesellschaft zur Förderung der angewandten Forschung e.V., 80686 München (DE)
(72) Inventor: KNAUER, Jan, Felix, 14476 Postdam (DE); KUBICK, Stefan, 14476 Postdam (DE); ZEMELLA, Anne, 14476 Postdam (DE)
(74) Representative: v. Bezold & Partner Patentanwälte - PartG mbB

(57) **Abstract**

The invention relates to a method for preparing functional membrane attached monooxygenases in cell-free systems which comprises at least the following steps:
a) providing a reaction medium comprising at least the following components:
a nucleic acid template coding for a Cytochrome P450 monooxygenase classified as EC 1.14.-.-, an RNApolymerase, a eukaryotic cell lysate containing ER-derived membrane vesicles which comprise NADPH-Cytochrom-P450-oxidoreductase (CPR), optionally heme or heme derivatives, in particular heme complexes selected from the group comprising or consisting of hematin and hemin;
b) performing a cell-free reaction to synthesize the monooxygenase protein, wherein the cell-free translation reaction is performed at a temperature in the range from 18 to 33°C.

The invention further relates to a reaction medium for preparing a functional Cytochrome P450 monooxygenase classified as EC 1.14.-.- in a cell-free system comprising at least the following components:
a nucleic acid template coding for a Cytochrome P450 classified as EC 1.14.-.-, an RNA polymerase, a eukaryotic cell lysate containing membrane vesicles which comprise NADPH-Cytochrom-P450-oxidoreductase (CPR), heme or heme derivatives, in particular heme complexes selected from the group comprising or consisting of hematin and hemin.

## Description

### Background of the invention

Cytochromes P450 (CYPs) are a group of monooxygenases that can be found in almost all kinds of organisms. The name derives from its characteristic light absorbance attribute that is due to the inherent heme group. In contrast to the soluble cytochromes that are involved in the respiratory chain, CYPs are membrane bound and can be found in different compartments of eukaryotic cells. A very prominent localization of CYPs is the surface of the endoplasmatic reticulum, especially in hepatocytes. In humans, CYPs are an integral part of liver-based phase-1 biotransformation, which is essential for the metabolization of multiple xenobiotics and drugs. Consequently, CYPs are important players during drug development and therefore these enzymes are implemented in diverse screening applications. Additionally, CYPs are used for the conversion of difficult to synthesize metabolites.

Due to their wide range in application, there is a growing demand for the production of CYPs, in research and in the drug discovery process. However, primary hepatocyte microsomes contain all variants of endogenous CYPs thereby impeding the isolation and analysis of specific CYPs, in the following also referred to as mono CYPs. The recombinant expression of CYPs has already been extensively investigated (Hausjell et al. 2018, Bioscience reports 38(2). DOI:10.1042/BSR20171290). However, especially eukaryotic CYPs were found difficult to produce. Restrictions originate from the complex co-factor requirements (the heme group) and the availability of a redox partner, the NADPH-Cytochrome P450 Oxidoreductase (CPR).

Generally, bacterial systems such as *Escherichia coli* are probably the most popular platform for recombinant protein expression, due to its straightforward handling and high growth rate. However, the synthesis of complex eukaryotic proteins like CYPs in such systems is unfavorable since they can only be expressed in a modified soluble form (Hausjell et al. 2018).

In contrast to prokaryotes like *E. coli,* yeast as well as higher eukaryotes like insect and mammalian cells, possess organelles like the endoplasmic reticulum and the golgi apparatus, enabling proper anchoring of membrane bound proteins like the CYPs. Further, the enable the achievement of various post-translational modifications of complex proteins.

It has been shown that the expression of functional CYPs is principally possible in genetically engineered yeast cell lines and the most successful system regarding the protein yield was described in a study about the expression of a plant originating CYP73A1 in S. *cerevisiae* (Schoch et al. 2003, Plant physiology 133 (3), 1198-1208). However, even this system suffered from rather low protein yields.

Besides expression in yeast cells, expression of CYPs in other eukaryotic cell lines, like *Sf*21 insect cells and CHO cells, was performed in a few studies (Lee et al. 1995, Archives of Biochemistry and Biophysics (319), 157-167; Ding et al. 1997, Archives of Biochemistry and Biophysics (348), 403-410; Augustin et al., 2013, Acta pharmacologica Sinica 34 (1), 146-156). The motivation of these studies, however, was not the isolation and characterization of a specific CYP enzyme, but rather the effects of its overexpression to the cells in different contexts. Consequently, the systems described therein are only partially suited for efficient production of CYPs in the respective cells lines used. At least from insect cell lines commercially available microsomes containing recombinant CYPs exist. However, they are relative expensive and only few CYP variants are available. Therefore, these microsomes allow studies on already well researched CYP variants but lack adaptability for the research on further variants and mutants.

Principally, cell-free systems would offer a number of advantages, for example they are exceedingly adaptable and would allow for a parallel screening application of multiple CYP variants and mutants. To date, however, no successful approach for the production of CYPs employing cell-free protein synthesis (CFPS) based on eukaryotic cell lysates has been reported. Even though first approaches on bacterial CYPs produced in *E. coli* based cell-free systems are published (Kwon et al. (2013), Biotechnology and bioengineering 110 (4), S. 1193-1200. DOI: 10.1002/bit.24785), there are no studies on eukaryotic, in particular human, CYPs produced in cell-free protein synthesis systems.

This might be due to the fact that - according to findings of the present inventors-the process for preparing suitable eukaryotic lysates for the production of Cytochrome P450 monooxygenases as well as the cell-free reaction itself must be carefully optimized for efficient synthesis.

For example, the lysis process must be neither too gentle nor too harsh to allow efficient protein synthesis performance but also preservation of endogenous microsomes. Non-functional or absent microsomes lead to deficient membrane protein synthesis with inactive CYPs. When using modified cell lines, the properties of the cell line used must be compatible with the lysate production protocol, especially the cultivation and lysis conditions. For the cell-free reaction, it is important to use the proper amounts of any additives and the proper synthesis temperatures, since conventional reaction temperatures for eukaryotic lysates have been found too high for the generation of functional Cytochrome P450 monooxygenases.

In view of this situation, the main object underlying the present invention is the provision of novel and improved means for cell-free synthesis of a range of functional Cytochrome P450 enzymes, which overcome or considerable alleviate the drawbacks of the prior art.

This object is achieved according to the present invention by providing the method for preparing functional membrane attached Cytochrome P450 monooxygenases classified as E.C. 1.14.-.- using a cell-free eukaryotic system according to claim 1 as well as the reaction medium for use in such a method according to claim 10. Additional aspects and preferred embodiments of the present invention are the subject of further claims.

### Description of the invention

The method of the present invention for preparing a functional membrane attached monooxygenase in cell-free systems comprises at least the following steps:
a) providing a reaction medium comprising at least the following components:
   a nucleic acid template coding for a Cytochrome P450 monooxygenase (CYP) classified as EC 114.-.- , an RNA polymerase, a eukaryotic cell lysate containing membrane vesicles which comprise NADPH-Cytochrom-P450-oxidoreductase (CPR), and optionally heme or heme derivatives, in particular heme complexes selected from the group comprising or consisting of hemin and hematin
b) performing a cell-free reaction to synthesize the monooxygenase protein, wherein the cell-free translation reaction is performed at a temperature in the range from 18 to 33°C.

Preferably, the reaction medium comprises a heme or heme derivative, in particular hemin or hematin. The heme or heme derivative should be added as freshly as possible and as late as possible in the reaction to avoid a failure or impairment of the protein synthesis reaction. Typically, the heme or heme derivative is added to a volume of the reaction mixture which represents at least 80% of the volume of the final reaction mixture and which already contains all the buffers required in the final reaction mixture. Depending on the synthesis performance, the heme concentration can be adjusted to ensure optimal synthesis efficiency. Generally, the heme concentration is in the range of from 1 µM to 100 µM, preferably from 1 µM to 25 µM, especially preferred about 5 µM.

Typically, the cell-free translation reaction is effected at a temperature in the range from 18 to 33°C, preferably in the range from 18 to 29°C, especially preferred in the range from 18 to 24°C.

The nucleic acid template coding for a Cytochrome P450 monooxygenase may be a mRNA template or a DNA template and preferably is a DNA template.

Particularly in insect cell-based systems, optimized workflows for linked CFPS (mRNA as template) as well as coupled CFPS (DNA as template) have already been established (e.g. Kubick et al. (2009) in Current topics in membranes, 63, 25-49).

The nucleic acid template may further comprises a nucleic sequence coding for a melittin signal peptide which has been proven beneficial for the translocation of various proteins in insect and mammalian CFPS (e.g., Stech et al. (2013) Journal of biotechnology 164 (2), S. 220-231. DOI: 10.1016/j.jbiotec.2012.08.020).

Advantageously, the cell-free preparation method according to the present invention is applicable to broad range of Cytochrome P450 monoxygenases which comprise a variety of heme containing enzymes of several enzyme classes (EC 1.14.13-99.-) that belong to the oxidoreductases.

In some preferred embodiments, the Cytochrome P450 monooxygenases classified as EC 1.14.-.- may be selected from the group comprising Cytochrome P450 1A2 (EC 1.14.14.1), Cytochrome P450 2B6 (EC 1.14.13.), and Cytochrome P450 3A4 (EC 1.14.14.).

The RNA polymerase used in the method according to the present invention is not especially limited. Preferably, the RNA polymerase is selected from the group comprising T3, T7 polymerase, and SP6 polymerase.

The eukaryotic cells, in particular insect cells or mammalian cells, used in the present invention are not especially limited. Principally, any eukaryotic cells used in the prior art for preparing cell lysates are suitable which are capable to provide cell lysates containing microsomes, i.e. endogenous ER-derived membrane vesicles, wherein the membrane vesicles contain NADPH-Cytochrom-P450-oxidoreductase (CPR).

Specifically, the insect cells or mammalian cells may be selected from the group which comprises *Spodoptera frugiperda* cells, CHO cells and K562 cells.

In a preferred embodiment of the claimed method, the ER-derived membrane vesicles in the reaction medium originate from the same cell line as the cell lysate and are typically provided by and contained in said cell lysate.

Microsomes generally allow protein translocation and posttranslational modifications of the respective target proteins such as N-glycosylations and were reported for several mammalian and insect cell based systems. Furthermore, the microsomal lumen is much more favorable for the formation of disulfide bonds compared to the bulk reaction medium outside the microsomes.

In order to provide a desired a desired target level of CPR within the microsomes it is possible to use either eukaryotic cells expressing endogenous CPR or eukaryotic cells modified to express or overexpress CPR. The stable modification of eukaryotic CHO cells with CPR has been shown earlier (Ding et al. (1997) Archives of Biochemistry and Biophysics 348 (2), S. 403-410. DOI: 10.1006/abbi.1997.0405). The desired CYP enzyme can be synthesized in the translationally active modified CHO-CPR lysate in a straightforward manner.

The preparation of cell lysates from the various eukaryotic cells mentioned above is well known in the art (e.g. Brodel, 2013 https://doi.org/10.1002/bit.25013) and, if desired, suitable modifications of known procedures can be effected by the skilled artisan.

In a closely related aspect, the present invention further relates to a reaction medium, in particular for preparing a functional Cytochrome P450 monooxygenase in a cell-free system, comprising at least the following components:
- a nucleic acid template coding for a Cytochrome P450 classified as EC 114.-.-
- anRNApolymerase
- a eukaryotic cell lysate, in particular derived from insect cells or mammalian cells, comprising membrane vesicles which comprise NADPH-Cytochrom-P450-oxidoreductase (CPR)
- heme or a heme derivative, in particular a heme complex selected from the group comprising or consisting of hematin and hemin.

Specifically, the insect cells or mammalian cells from which the eukaryotic cell lysate is derived may be selected from the group which comprises *Spodoptera frugiperda* cells, CHO cells and K562 cells.

In a more specific embodiment of the invention, the reaction medium comprises hemin or hematin in a concentration in the range of from 1 µM to 100 µM, preferably from 1 µM to 25 µM, in particular about 5 µM.

### Brief description of the Figures

**Fig. 1** shows analytical data of cell-free synthesized cytochromes CYP1A2, CYP2B6 and CYP3A4: (A) SDS-PAGE and autoradiography; (B) protein yields.
**Fig. 2** shows the activity of synthesized CYP1A2, CYP2B6 and CYP3A4.
**Fig. 3** shows the effect of hematin concentration in the reaction medium on the yield of active CYP3A4 (expressed as relative activity).

The present invention is further illustrated by the following specific but non-limiting examples.

### EXAMPLE 1

### Synthesis and characterization of CYP1A2, CYP2B6 and CYP3A4

Mammalian cell lysates, derived from CHO K1 cells or CPR-expressing modified CHO K1 cells, were utilized for cell-free synthesis of three exemplary monooxygenases, CYP1A2, CYP2B6 and CYP3A4.

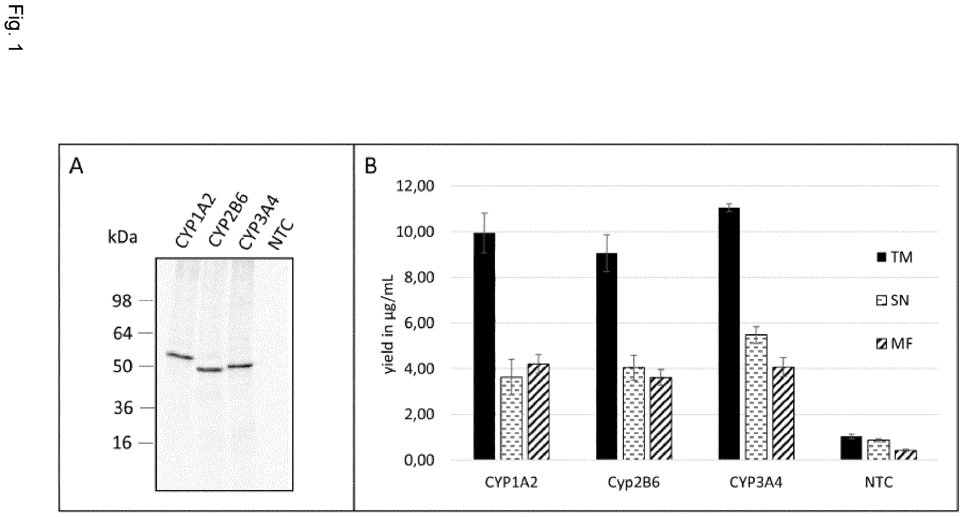

Plasmids, suitable for CFPS, coding for the respective CYP, were applied as template. T7 RNA polymerase, amino acids, an energy regeneration system and other supplements were added to the translationally active lysates according to Stech et al. (Journal of biotechnology 164 (2), 220-231 (2012)) with the additional supplementation of 5 µM heme to the reaction and a reaction temperature of 24°C. Autoradiography revealed the successful cell-free synthesis of CYP1A2, CYP2B6 and CYP3A4 (Fig. 1A). The synthesis efficiency was quantified by scintillation counting after TCA precipitation of the samples (Fig. 1B).

Fig. 1 shows analytical data of cell-free synthesized CYP1A2, CYP2B6 and CYP3A4 obtained as follows: The translation mixture was supplemented with ¹⁴C-Leucine which allows qualitative and quantitative validation of the protein synthesis process. Cell-free synthesis of the target enzymes was detected in the translation mixture through SDS-PAGE (10%) and subsequent autoradiography allowing for the detection of radioactively labeled proteins (A) Cell-free synthesis of CYPs was compared to no-template controls (NTC). TCA precipitation and subsequent scintillation counting determined the protein yield of produced labeled proteins (B). Besides the translation mix (TM), the microsomal fraction (MF) and the supernatant (SN) after 16.000 x g centrifugation for 10 min was analyzed. The yield determination was conducted in triplicates.

The activity of CYPs in the translation mix as well as in the microsomal fraction can additionally be detected by luciferase assays. As an example, three different CYPs were analysed using different luciferase based assays by Promega.

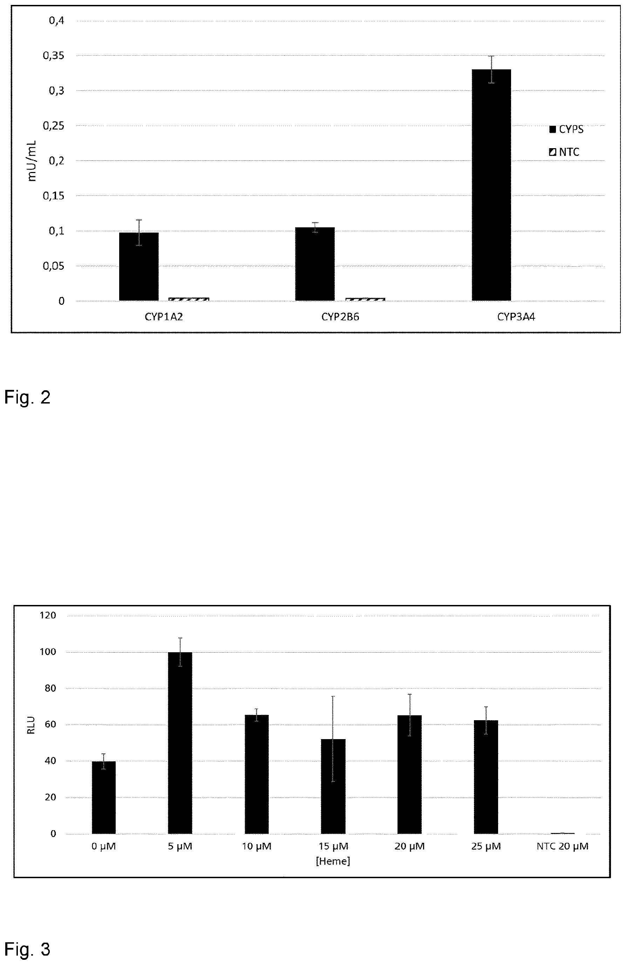

Fig. 2 shows the activity of cell-free synthesized CYP1A2, CYP2B6 and CYP3A4: The microsomal fraction was obtained after centrifugation of the translation mixture at 16.000 x g. The assay was performed in duplicates. A no-template control (NTC) shows the background of the assay. A calibration was performed to estimate the volume activity. CYP activity could be determined for all tree CYPs in the microsomal fraction. In no-template controls, almost no CYP activity could be detected.

CYPs are membrane associated proteins, but in contrast to most trans-membrane proteins they are only N-terminal anchored in the membrane and have a partially lipophilic surface that is oriented to the membrane. The translocation process is therefore different to other membrane proteins that have been produced already successfully by CFPS. Localization and the influence of signal sequences are therefore an important issue for the cell free synthesis of CYPs. The localization of the cell-free produced CYPs was analyzed using confocal laser scanning microscopy. For this purpose, templates for CYP3A4-eYFP fusion proteins were generated. Additionally, a template containing a melittin signal sequence (Mel-CYP3A4-eYFP) was generated. Both templates were used for the cell-free protein synthesis in the modified CHO-lysates and led to a co-localization of the target protein with the microsomes (data not shown).

### Detailed experimental procedures

### Design of DNA Templates

Templates for the synthesis of CYPs in cell-free systems were generated by Biocat GmbH. The protein encoding sequence (Gene number 714916-1/2/3, 724709-12) was integrated in a pUC57-1.8k-vector backbone comprising further regulatory factors for CAP-independent protein synthesis by using a Cricket paralysis virus-IRES (Brodel et al. 2013; PloS one 8 (12), e82234.DOI: 101371/journal.pone.0082234).

### Cell fermentation, lysis and lysate processing

Suspension adapted Chinese Hamster Ovary cells (CHO-K1) were routinely cultivated in ProCHO5 medium (Lonza Group AG, Basel, Switzerland) supplemented with 6 mM L-alanyl-L-glutamine (Merck, Darmstadt, Germany). CHO suspension cells were cultured in unbaffled flasks (Corning, New York, USA) at 37 °C and 5 vol-% CO₂ at 100 rpm on an orbital shaker. CHO cells were grown in suspension cultures in shaking flask to a maximal volume of 500 mL and in fermenters above a volume of 500 mL. CHO cells were harvested at a density of approximately 4 × 10⁶ cells/mL. Cell washing, lysis and lysate processing were performed as described earlier (Brodel et al. (2014), Biotechnology and bioengineering 111(1), 25-36; Thoring et al. (2016); PloS one 11 (9), e0163670.DOI: 101371/journal.pone.0163670). During incubation in the fermenter, viability, oxygen concentration, pH and cell density were monitored.

CHO-K1 modified variants expressing human CPR (CHO-CPR) or a co-expression of human CPR and CYP3A4 (CHO-CPR/CYP3A4) (kindly provided by Prof. Dr. Küpper, BTU Cottbus-Senftenberg, Germany) were cultivated similar to CHO-K1 cells. Blasticidin (Biovision GmbH, Ilmenau, Germany) (3 µg/mL, resistance of the CPR expression vector) or Blasticidin and Zeocin (Abcam, Cambridge, UK) (300 µg/mL, resistance of the CYP3A4 expression vector) were added to the culture medium, to maintain the expression of human CPR or CPR/CYP3A4 in the corresponding CHO cell lines. The lysis process for the generation of translationally active lysates was the same as for wild type CHO-K1 cells.

### Cell-free protein synthesis

Synthesis of proteins in translationally active lysates derived from cultured Chinese hamster ovary (CHO) cells and its modified variants CHO-CPR and CHO-CPR/CYP3A4 cells, was performed in batch based systems as previously described (Thoring et al. 2016). Accordingly designed plasmids, suitable for CFPS, coding for the CYP of interest, were applied as template. T7 RNA Polymerase, amino acids, an energy regeneration system and other supplements were added to the translationally active lysates with the additional supplementation of 5 µM heme (porcine) (Alfa Aesar Haverhill, Massachusetts, USA) to the reaction and a reaction temperature of 24 °C despite elsewise noted. For the isolation of the microsomes the translation mixture (TM) was centrifuged at 16.000 x g for 10 minutes at 4 °C. The pellet was resuspended in the same volume PBS to receive the microsomal fraction (MF). The microsomal fraction comprises the endogenous microsomes derived from the endoplasmic reticulum including the *de novo* synthesized membrane bound proteins.

### Protein yield determination

To validate successful cell-free protein synthesis, radioactive labeling of *de novo* synthesized proteins with ¹⁴C-leucine was performed enabling qualitative characterization by autoradiography and quantitative analysis through scintillation counting as described earlier (Stech et al. 2012).

### SDS PAGE and Autoradiography

Sodium dodecyl sulfate polyacrylamide gel electrophoresis (SDS-PAGE) and autoradiography were used to analyze homogeneity and molecular weight of *in vitro* translated proteins. 45 µL water were added to 5 µL of the sample and precipitated with 150 µL ice cold acetone at 4 °C for at least 15 min. Precipitated proteins were pelleted at 16,000 x g for 10 min at 4 °C. Protein pellets were dried for 1 h at 45 °C and re-suspended in 20 µL LDS sample buffer. The samples were loaded onto 10 % SDS-PAGE gels. SDS-PAGE was performed at 150 V for 1 h. The gels were stained for 1 h using SimplyBlue - SafeStain, and destained in water over night. The gels were dried (Unigeldryer) for 70 min at 70 °C. The dried gels were put on a phosphor screen for at least three days. Radiolabeled proteins were visualized on a phosphor-imager system.

### Western blot

Western blotting and subsequent antibody detection were used for the identification of endogenous and *de novo* synthesized CYP3A4 and CPR in the translation mixture of the cell-free synthesis reaction. SDS PAGE was performed like described above. Proteins were blotted on a PVDF membrane with an iBlot device (Thermo Fisher Scientific, Waltham, Massachusetts, USA). The membrane was washed three times with TBS and subsequently blocked with 2 % Bovine Serum Albumin (BSA) (Carl Roth GmbH + Co. KG, Karlsruhe, Germany) over night at 4 °C. After three washing steps with TBS/T, the membrane was incubated with the primary antibody in a concentration of 0.4 µg/mL in 2 % BSA for three hours at room temperature. The blot was washed three times with TBS/T and incubated with a secondary Horse Radish Peroxidase (HRP) linked antibody at a final concentration of 0.5 µg/mL in 2 % BSA at room temperature for one hour. Three final washing steps in TBS/T were performed. Chemiluminescent signals were detected after incubation with ECL detection reagent. The primary antibody used for the detection of CPR was "CYPOR (F-10): sc-25270" (Santa Cruz Biotechnology, Dallas, Texas, USA), the primary antibody used for the detection of CYP3A4 was "CYP3A4 (HL3): sc-53850" (Santa Cruz Biotechnology, Dallas, Texas, USA).

### CPR activity assay

CPR activity was determined by "Cytochrome P450 Reductase Activity Assay Kit" (Abcam, Cambridge, UK) using the manufacturers protocol. The activity was determined directly in the translationally active lysate of wild type (wt) CHO cells and CHO-CPR cells. Additionally the microsomal fraction was isolated as described previously. The activity was quantified using a calibration curve that was generated with supplements supplied by the kit.

### CYP activity assays

For CYP activity measurement,"P450-Glo^{™} Assays" (Promega, Madison, Wisconsin, USA) were used. CYP1A2 activity was detected by Luciferase-ME turnover (V8772), CYP2B6 was detected by Luciferin-2B6 turnover (V8321) and CYP3A4 was detected by Luciferin-IPA turnover (V9001). The CYP reaction was performed according to the Promega "P450-Glo^{™} Assays" protocol except the CYP reaction time was prolonged to 1 h unless noted otherwise. The CYP reaction temperature was set at 37 °C. The NADPH Regeneration System (V9510) was used for the supply of NADPH during the assay. Three control approaches were performed, one with buffer control, one designed as no template control and one control, using human liver cell microsomes (Gibco^{™} Human Microsomes, 50 Donors) (Thermo Fisher Scientific, Waltham, Massachusetts, USA) as positive control. Human microsomes were tested at a final concentration of 0.4 mg/mL. If not otherwise noted 5 µL of the microsomal fraction of the cell-free reaction were applied as samples to the activity assay. For response condition adjustments, CYP activities were usually expressed as percentage of the highest CYP activity during the assay. For CYP activity quantification, a standard curve was prepared using beetles luciferin (Promega, Madison, Wisconsin, USA) according to the attached protocol.

### Indirect substrate screening

Luciferase-based assays were also used as a preliminary screening procedure for the turnover of various potential CYP substrates. The substrates testosterone (Sigma-Aldrich Chemie GmbH, Taufkirchen, Germany), midazolam (Sigma-Aldrich Chemie GmbH, Taufkirchen, Germany), efavirenz (Fisher Scientific GmbH, Schwerte, Germany), and phenacetin (Fisher Scientific GmbH, Schwerte, Germany) were solved in a concentration of 3 mM in 100 % methanol. As a control for a sterol that is not known to be turned over by CYPs, cholesterol was prepared in the same way. Cell-free CYP synthesis and isolation of the microsomal fraction was performed as described above. The luciferase based CYP activity assay was performed using 5 µL of the microsomal fraction of the cell-free synthesis of CYP1A2, CYP2B6 and CYP3A4. A final concentration of 200 µM of the analyzed substrate was added to the CYP reaction in parallel to the specific luciferase substrate. A vehicle control with methanol was performed to exclude an influence of the solvent. Changes in the turnover of the luciferase product indicate an interaction of the test substrate with the tested CYP. Changes in luminescence signal were expressed in percentage with reference to the result from the batch without addition of a substrate.

### EXAMPLE 2

### Effect of heme concentration in the reaction medium on the yield of active CYP3A4

Heme is a cofactor of CYPs and is therefore mandatory for its function. Adaption of the amount of supplemented heme to the cell free reaction is therefore necessary. Heme was supplemented in different concentrations to different batches of the cell-free reaction. The CYP activity in the MF was determined by the Luciferase based CYP3A4 activity assay (triplicate analysis). A heme concentration of 5 µM resulted in the highest CYP3A4 activity, which was more than twofold higher compared to the control without supplementation (Figure 3).

The supplementation of higher concentrations of heme results in equally 60 % reduced activity compared to the 5 µM heme supplemented sample. The concentration of 5 µM heme was used in all subsequent batches.

## Claims

1. A method for preparing a functional membrane attached monooxygenase in cell-free systems comprising at least the following steps:
a) providing a reaction medium comprising at least the following components:
a nucleic acid template coding for a Cytochrome P450 monooxygenase classified as EC 1.14.-.-
anRNApolymerase
a eukaryotic cell lysate containing ER-derived membrane vesicles which comprise NADPH-Cytochrom-P450-oxidoreductase (CPR)
optionally heme or heme derivatives, in particular heme complexes selected from the group comprising or consisting of hematin and hemin;
b) performing a cell-free reaction to synthesize the monooxygenase protein, wherein the cell-free translation reaction is performed at a temperature in the range from 18 to 33°C.

2. The method according to claim 1, wherein the eukaryotic cell lysate is derived from insect cells or mammalian cells.

3. The method according to claim 2, wherein the insect cells are *Spodopterafrugiperda* cells or the mammalian cells are selected from CHO and K562 cells.

4. The method according to anyone of claims 1 to 3, wherein the eukaryotic cell lysate is derived from eukaryotic cells modified to express a target level of NADPH-Cytochrom-P450-oxidoreductase (CPR).

5. The method according to anyone of claims 1 to 4, wherein the nucleic acid template coding for a Cytochrome P450 monooxygenase classified as EC 1.14.-.- is a DNA template.

6. The method according to anyone of claims 1 to 5, wherein membrane vesicles originate from the same cell line as the cell lysate.

7. The method according to any one of claims 1 to 6, wherein the cell-free translation reaction is performed at a temperature in the range from 18 to 29°C, preferably in the range from 18 to 24°C.

8. The method according to any one of claims 1 to 7, wherein the Cytochrome P450 monooxygenase classified as EC 1.14.-.- is selected from the group comprising Cytochrome P450 1A2 (EC 1.14.14.1), Cytochrome P450 2B6 (EC 1.14.13.), and Cytochrome P450 3A4 (EC 1.14.14.).

9. The method according to any one of claims 1 to 8, wherein the RNA polymerase is selected from the group comprising T3, T7 polymerase, and SP6 polymerase.

10. A reaction medium for preparing a functional Cytochrome P450 monooxygenase classified as EC 1.14.-.- in a cell-free system comprising at least the following components:
a nucleic acid template coding for a Cytochrome P450 classified as EC 1.14.-.- anRNApolymerase
a eukaryotic cell lysate containing membrane vesicles which comprise NADPH-Cytochrom-P450-oxidoreductase (CPR)
heme or heme derivatives, in particular heme complexes selected from the group comprising or consisting of hematin and hemin.

11. The reaction medium according to claim 10 or the method according to any one of claims 1 to 9, wherein the reaction medium comprises the heme or heme derivative in a concentration in the range of from 1 µM to 100 µM, preferably from 1 µM to 25 µM, in particular about 5 µM.
